# EUROPEAN PATENT SPECIFICATION

(11) **EP 1 943 204 B1**
(45) Date of publication and mention of the grant of the patent: **02.01.2013**
(21) Application number: 06836872.9
(22) Date of filing: 03.11.2006
(51) Int. Cl.: C07C 17/266, C07C 17/269, C07C 21/18

(54) **METHOD FOR PRODUCING FLUORINATED ORGANIC COMPOUNDS**
VERFAHREN ZUR HERSTELLUNG FLUORIERTER ORGANISCHER VERBINDUNGEN
PROCEDE DE PRODUCTION DE COMPOSES ORGANIQUES FLUORES

(30) Priority: 03.11.2005 US 733379 P
(43) Date of publication of application: 16.07.2008
(73) Proprietor: Honeywell International Inc., Morristown, NJ 07960 (US)
(72) Inventor: MUKHOPADHYAY, Sudip, Williamsville, NY 14221 (US); NAIR, Haridasan, Williamsville, NY 14221 (US); TUNG, Hsueh Sung, Getzville, NY 14068 (US); VAN DER PUY, Michael, Amherst, NY 14221 (US); JOHNSON, Robert, Lancaster, NY 10486 (US); MERKEL, Daniel, C., West Seneca, NY 14224 (US)
(74) Representative: Hucker, Charlotte Jane
(86) International application number: PCT/US2006/042935
(87) International publication number: WO 2007/056127

(56) References cited:
- US-A- 2 931 840

## Description

### BACKGROUND OF INVENTION

### (1) Field of Invention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such tetrafluoropropenes (including 2,3,3,3-tetrafluoro-1-propene (HFO-1234yf) and 1,3,3,3-tetrafluoro-1-propene (HFO-1234ze)) have been disclosed to be effective refrigerants, fire extinguishants, heat transfer media, propellants, foaming agents, blowing agents, gaseous dielectrics, sterilant carriers, polymerization media, particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. Although this appears to be a relatively high-yield process, for commercial scale production the handling of hydrogen gas at high temperature raises difficult safety related questions. Also, the cost of producing hydrogen gas, such as building an on-site hydrogen plant, can be prohibitive in many situations.

U.S. Pat. No. 2,931,840 (Marquis) describes a method for making fluorine containing olefins by pyrolysis of methyl chloride and tetrafluoroethylene or chlorodifluoromethane. This process is a relatively low yield process and a relatively large percentage of the organic starting material is converted in this process to unwanted and/or relatively unimportant byproducts, including a sizeable amount of carbon black. The carbon black is not only unwanted, it tends to deactivate the catalyst used in the proces.

The preparation of HFO-1234yf from trifluoroacetylacetone and sulfur tetrafluoride has been described. See Banks, et al., Journal of Fluorine Chemistry, Vol. 82, lss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Catalyzed hydrogen reduction reactions have been disclosed for the preparation of fluorinated C3 hydrocarbons in U.S. Patent No. 5,545,777. The patent describes the reaction as being one in which a compound of formula (1)

C3HₐCl_{b}F_{c} (1)

is converted by catalyzed hydrogen reduction to a compound of formula (2)

C₃Hₐ₊ₓCl_{b-y}F_{c-z} (2)

where a, b, c, x, y and z are integers satisfying the following conditions: a≥0, b≥1, c ≥2, x ≥1, y ≥1, z ≥0, a+b+c=8, x=y+z, b-y≥0, and c-z≥2. Since the reactions disclosed in this patent require a reaction product in which a+b+c=8 and that x=y+z, the disclosed reaction product does not include C3 olefins, which as mentioned above have been found to be desirable for use in many important applications.

Notwithstanding prior teachings, applicants have come to appreciate a continuing need for methods for efficiently preparing certain hydrofluorocarbons, particularly halogenated olefins such as the fluorinated propenes, including HFO-1234yf.

### SUMMARY OF THE INVENTION

Applicants have developed a method for producing HFO-1234 yf, which comprises contacting CF₃Cl with CH₃CF₂Cl, wherein the contacting step is carried out in the presence of a metal-based catalyst.

In preferred embodiments, the contacting step proceeds conditions effective to preferably convert at least 50%, more preferably at least 70%, and even more preferably at least 90%, of the CH₃CF₂Cl it is also generally preferred that said converting step produces a reaction product having at least 20% selectivity, more preferably at least 40% selectivity and even more preferably at least 70% selectivity to polyfluorinated C2 - C6 olefins, more preferably polyfluorinated propenes, and even more preferably tetrafluoropropenes. In highly preferred embodiments said converting step produces a reaction product having at least about 20% selectivity, more preferably at least about 40% selectivity and even more preferably at least about 70% selectivity HFO-1234yf.

In certain preferred embodiments, the converting step comprises reacting CH₃CF₂Cl with CF₃Cl in the gas phase, in the liquid phase, or a combination of these.

### DETAILED DESCRIPTION OF PREFERRED EMBODIMENTS

One beneficial aspect of the preferred form of the present invention is that it enables the production of desirable fluoroolefins, preferably C3 fluoroolefins, and in particular HFO-1234 yf, using relatively high conversion and high selectivity reactions. In addition, the preferred embodiments of the present methods provide reactions with relatively high yield and which are capable of obtaining relatively long catalyst life.

Furthermore, the present method permits the production of the desirable fluoroolefins from relatively attractive starting materials, which are relatively easy to handle, and/or are generally readily available In commercial quantities, and/or can be easily produced from other readily available materials.

In preferred embodiments, the CH₃CF₂Cl:CF₃Cl mole ratio of the feed(s) to the reactor are from 1:1 to 4:1 and even more preferably from 1.5:1 to 2.5:1. In many preferred embodiments the contacting step takes place in a single vessel and is considered to be a one-step reaction. We have surprisingly discovered that in many embodiments the presence of air, or other oxygen-containing material, in the feed to the reactor is highly preferred. Without necessarily being bound by to any particular theory of operation, it is believed that advantage is achieved in such embodiments because carbon is detrimentally deposited on the catalyst surface in the absence of air or its principal components. Without intending to be bound by or limited to any particular theory of operation, it is believed that in certain embodiments the reaction proceeds via the formation of CF₃CF₂CH₃ (HFC-245cb), which is quickly or substantially instantaneously dehydrofluorinated to HFO-1234yf under the preferred reaction conditions of the present invention. Another aspect of this not limiting theory is that the mechanism of this embodiment of the present invention is understood and believed to be that the reaction is initiated by the heterolytic clevage of the C-Cl bonds in chlorodifluoroethane (HCFC-142b) and similar compounds, such as chlortrifluoromethane (CFC-13).

In other embodiments, it is preferred that the contacting step comprises oxidative coupling of the starting materials. The reaction product includes, preferably in high yield and high selectivity, tetrafluoropropene(s), and in particular HFO-1234yf. As with the particular embodiments mentioned above, it is desirable and highly preferred in certain situations to include air or its principal components in the feed stream to the reactor in order to enhance catalyst life and effectiveness.

It is generally preferred that this reaction step be carried out in the gas phase, or in some embodiments possibly a combination of liquid/gas phases, and it is further contemplated that the reaction can be carried out batch wise, continuous, or a combination of these.

Thus, it is contemplated that the preferred contacting steps may be preformed using a wide variety of process parameters and process conditions in view of the overall teachings contained herein. However, it is preferred in certain embodiments that the contacting step includes a reaction step comprising a gas phase reaction in the presence of a metal-based catalyst, which can be supported on carbon or unsupported, and more preferably a nickel based catalyst (such as nickel mesh) and/or palladium-based catalyst, including palladium on carbon catalysts. It is contemplated, although not necessarily preferred at the present time, that other catalysts, such as antimony-based catalysts (including SbF₃, SbF₅, and partially flourinated SbCl₃ or SbCl₅), aluminum-based catalyst (such as AlCl₃), iron-based catalyst (such FeCl3), including such catalysts on a carbon or other appropriate support, may be used in accordance with the present invention. It is expected that many other catalysts may be used depending on the requirements of particular embodiments, and of course, two or more of any of these catalysts, or other catalysts not named here, may be used in combination.

The gas phase addition reaction may be conducted, for example, by introducing a gaseous form of CF₃Cl and CH₃CF₂Cl, into a suitable reaction vessel or reactor. Preferably the vessel is comprised of materials which are resistant to corrosion, such as Hastelloy, Inconel, Monel and/or fiuoropolymers iinings. Preferably the vessel contains catalyst, for example a fixed or fluid catalyst bed, packed with a suitable addition catalyst, with suitable means to heat and/or cool the reaction mixture to the desired reaction temperature.

While it is contemplated that a wide variety of reaction temperatures and pressures may be used, depending on relevant factors such as the catalyst being used and the most desired reaction product, it is generally preferred that at least a portion of the reaction step is carried out at a reaction temperature of from 5 °C to 1000°C, and even more preferably from 400°C to 800°C, and even more preferably from 450°C to 700°C for reactors which are preferably maintained at a pressure of from 7 to 342 kPa (1 to 1500 psig), more preferably from 7 to 345 kPa (1 psig to 50 psig), and even more preferably from 7 to 69 kPa (1 to 10 psig).

In preferred embodiments the conversion of the CH₃CF₂Cl compound is preferably at least 15%, more preferably at least 40%, and selectivity to tetrafluoropropene compounds is preferably at least 1%, more preferably at least 5%, and even more preferably at least 20%.

### A. CONTACTING STEP - FORMULA (II) COMPRISES CH₃CF₂CL

Applicants have discovered that CH₃CF₂Cl, can be reacted with CF₃Cl, to synthesize tetrafluorinated compounds and in particular, CF₃CF=CH₂ (HFO-1234yf). In certain preferred embodiments, the contacting step includes a reaction of the type in which a certain flow of the CH₃CF₂Cl, such as about 50 sccm of CH₃CF₂Cl, together with a flow of the CF₃Cl, such as about 20 sccm of CF₃Cl, is introduced together with a flow of air (e.g., about 20 sccm) into a reaction vessel, which may include a pre-heater operating at a relatively elevated temperature, for example, from 400°C to 800°C (preferably in certain embodiments at about 450°C) containing the appropriate amount and type of catalyst in accordance with the present invention (for gas flows of the quantity mentioned in this paragraph, a bed of about 50 cc of catalyst in a 25mm (1-inch) Monel reactor at about 675°C). In certain embodiments, the effluent gas mixtures from the reactor may be passed through a 20% aqueous KOH solution to neutralize any HF formed during the reaction to KF and H₂O.

### CATALYST PREPARATION

Certain preferred methods for catalyst preparation are described below, and the catalysts prepared in accordance with this description were used in and are referred to in the experiments described in the examples which follow.

### Catalyst A

About 50 cc Ni-mesh is charged in the reactor and then reduced with 20 sccm of H₂ at about 650°C for about 6 hours. This pre-reduced catalyst is preferably used in certain embodiments of the reaction in accordance with the present invention.

### Catalyst B

About 50 cc Ni-mesh was used as the catalyst without H₂ pretreatment.

### Catalyst C

From 1.5 gm to 2.0 gm of Ni (II) hexafluoroacetylacetonate hydrate was dissolved in about 200 cc of methanol at about 55°C and then 50 cc of dried activated carbon 3mm; 1/8-inch cylinders) was gradually mixed with the precursor solution. The methanol was evaporated slowly under vacuum at room temperature and then dried in an oven under vacuum at about 100°C for 10 hours.

The dried mass was then charged into the 25 mm (1-inch) Monel reactor and then reduced with 20 sccm of H₂ at 550°C for 6 hours and then 2 hours at 650°C. The catalyst was finally then calcined under 20 sccm of N₂ at 700°C for about ½ hour.

### Catalyst D

About 5 gm of La₂NiO₄ was prepared using the citric acid complexing method. Stoichiometric amounts of nickel and lanthanum nitrates were dissolved in an aqueous solution. After a small excess of citric acid was added, the solution was evaporated by vigorously stirring at about 70°C to generate viscous syrup. To this syrup was added about 50 ml of methanol and about 50 cc of dried activated carbon. The resultant material was then dried in an oven at about 180°C for about 1 hour, followed by calcinations at 500°C for 2 hours and then at about 900°C for about 6 hours. The catalyst was finally reduced with about 25 sccm of H₂ at about 750°C for 8 hours.

### Catalyst E

Pd/C was prepared using 3 gm of Pd (II) acetylacetonate as the precursor, and then the procedure used to prepare catalyst C was used.

### Catalyst F

Powdered BaO was obtained from Aldrich and used after drying at about 450°C under about 20 sccm of N₂ for about 12 hours.

### Catalyst G

Powder 50 cc BaO was dispersed in an anhydrous solvent and then added a colloidal solution of about 1.3 gm of Ni in methanol to the mixture. The solvent was evaporated slowly under vacuum at room temperature and then dried in an oven under vacuum at about 100°C for 10 hours. The dried mass was then charged into a 25 mm (1-inch) Monel reactor and then dried at about 550°C for 6 hour and then calcined under 20 sccm of N₂ at 700°C for % hour.

### Catalyst H

About 50 cc of powder CaO was dispersed in an anhydrous solvent, and then a colloidal solution of about 1.3 gm of Ni in methanol was added to the mixture. The solvent was evaporated slowly under vacuum at room temperature and then dried in an oven under vacuum at 100°C for 10 hours. The dried mass was then charged into a 25 mm (1-inch) Monel reactor and then dried at 550°C for 6 hours and then calcined under about 20 sccm of N₂ at 700°C for about ½ hour.

### Catalyst I

About 50 cc of powder MgO was dispersed in an anhydrous solvent and then a colloidal solution of about 1.3 gm of Ni in methanol was added to the mixture. The solvent was evaporated slowly under vacuum at about room temperature and then dried in an oven under vacuum at about 100°C for about 10 hours. The dried mass was then charged into the 25 mm (1-inch) monel reactor and dried at 550°C for 6 hours and then calcined under 20 sccm of N₂ at 700°C for about ½ hour.

### EXAMPLES - SECTION A

Table 01 below shows results from experiments using as a first combination of reactants CH₃CF₂Cl and C₂F₆ (comparative example) and a second combination of reactants comprising CH₃CF₂Cl and CF₃Cl under substantially identical conditions. The reaction between CF₃Cl and CH₃CF₂Cl gave about 16 mol% CF₃CF=CH₂ (HFO-1234yf) at a CH₃CF₂Cl conversion level of about 72%. For reactions in which CF₃Cl was replaced by C₂F₆, only trace amount of HF)-1234yf were obtained. It was also learned that in the presence of air, CO₂ was one of the major byproducts, whereas carbon black was one of the main byproducts in the absence of air or its principal constituents.

**Table A1**

| **Effect of different starting materials on the R1234yf production rate^{a}** | | | | | |
|---|---|---|---|---|---|
| Reaction | T, °C | P, (psig) kPa | Conv. mol % | R1234yf mol% | Byproducts mol% |
| CH₃CF₂Cl +CF₃Cl+ Air | 675 | (2.6) 17.9 | 72 | 16 | CO₂(20%); C (5%); R1132 (12%); R143a(8%); R1131(12%); R134a(3%); R133a(2%);R22 (1%); R1130 (4%); R40 (5%); R1243 (1%); Unknown (11%) |
| CH₃CF₂Cl + C₂F₆ + Air * | 675 | (2.8) 19.3 | 57 | 1.5 | CO₂(42%); C(10%); R115a (15%); R114a(21%); R22 (2%); R1112a (1%); R113a(1%); R123a (2%), Unknown(4.5%) |

| | | | | | |
|---|---|---|---|---|---|
| **^{a}Reaction conditions: CH₃CF₂Cl, 50 SCCM; CF₃Cl, 20 SCCM; Air, 15 SCCM; Catalyst, G; Amount of catalyst, 50 cc; Conversion is the ratio of moles of CFC converted and moles of CFC taken initially x 100;1234yf % = Moles of CFC converted to 1234yf/total moles of CFC taken initially x 100** * comparative example | | | | | |

Table A2 shows the effect of process parameters such as reaction temperature, pressure, flow rates, and composition of catalysts on the reaction. The highest conversion to the product HFO-1234yf (that is, about 16%) was obtained using the conditions given In Table 2, Run 2 using Catalyst G.

**Table A2 Reaction of CH₃CF₂Cl and CF₃Cl in the presence of Air and catalyst^{a}**

| Run | T, °C | P, (psig) kPa | CH₃CF₂Cl, SCCM | CF₃Cl, SCCM | Air, SCCM | Cat | Conv. of CH₃CF₂Cl, mol % | Conv. to 1234yf, mol% |
|---|---|---|---|---|---|---|---|---|
| 1 | 600 | (3) 21 | 50 | 20 | 15 | G | 57 | 7 |
| 2 | 675 | (3) 21 | 50 | 20 | 15 | G | 72 | 16 |
| 3 | 700 | (3) 21 | 50 | 20 | 15 | G | 83 | 12 |
| 4 | 675 | (10) 69 | 50 | 20 | 15 | G | 73 | 14 |
| 5 | 675 | (15) 103 | 50 | 20 | 15 | G | 75 | 12 |
| 6 | 675 | (3) 21 | 30 | 20 | 15 | G | 62 | 9 |
| 7 | 675 | (3) 21 | 70 | 20 | 15 | G | 53 | 16 |
| 8 | 675 | (3) 21 | 50 | 20 | 15 | G | 68 | 16 |
| 9 | 675 | (3) 21 | 50 | 40 | 15 | G | 63 | 16 |
| 10 | 675 | (3) 21 | 50 | 20 | 5 | G | 53 | 9 |
| 11 | 675 | (3) 21 | 50 | 20 | 40 | A | 57 | 3 |
| 12 | 675 | (3) 21 | 50 | 20 | 15 | B | 49 | 1 |
| 13 | 675 | (3) 21 | 50 | 20 | 15 | C | 80 | 9 |
| 14 | 675 | (3) 21 | 50 | 20 | 15 | D | 83 | 11 |
| 15 | 675 | (3) 21 | 50 | 20 | 15 | E | 57 | 0 |
| 16 | 675 | (3) 21 | 50 | 20 | 15 | F | 68 | 6 |
| 17 | 675 | (3) 21 | 50 | 20 | 15 | H | 58 | 8 |
| 18 | 675. | (3) 21 | 50 | 20 | 15 | I | 84 | 12 |

| | | | | | | | | |
|---|---|---|---|---|---|---|---|---|
| **^{a}Catalyst A is Ni-mesh reduced; B is Ni-mesh; C is Ni/C; D is La₂NiO₄; E is Pd/C; F is BaO; G is Ni/BaO; H is Ni/CaO; I is Ni/MgO** | | | | | | | | |

Without intending to be bound by her to any particular theory, it is believed that the reaction mechanism is as follows:

CH₃CF₂Cl ↔ CH₃CF₂ ● + Cl●

CF₃Cl ↔ CF₃● + Cl●

CF₃CF₂● + CH₃● ↔ CF₃CF₂CH₃

CF₃CF₂CH₃ ↔ CF₃CF=CH₂ + HF

Cl● + Cl● ↔ Cl₂

## Claims

1. A process for the preparation of 2,3,3,3-tetrafluoro-1-propene (HFO-1234yf), the process comprising contacting CF₃Cl with CH₃CF₂Cl, said contacting step being carried out in the presence of a metal-based catalyst.

2. The process of claim 1, wherein the molar ratio of CH₃CF₂Cl:CF₃Cl is in the range from 1:1 to 4:1.

3. The process of claim 2, wherein the molar ratio of CH₃CF₂Cl:CF₃Cl is in the range from 1.5:1 to 2.5:1.

4. The process of any preceding claim, wherein the metal-based catalyst is selected from nickel-based catalysts, palladium-based catalysts, antimony-based catalysts, aluminium-based catalysts and iron-based catalysts.

5. The process of any preceding claim, wherein said contacting step is effective to convert at least 50% of said CH₃CF₂Cl.

6. The process of any preceding claim, wherein said contacting step is effective to convert at least 90% of said CH₃CF₂Cl.

7. The process of any preceding claim, wherein said contacting step is effective to produce a reaction product having at least 20% selectivity to HFO-1234 yf.

8. The process of claim 7, wherein said contacting step is effective to produce a reaction product having at least 70% selectivity to one or more tetrafluoropropenes.

9. The process of any preceding claim, wherein said contacting step is effective to produce a reaction product having at least 70% selectivity to HFO-1234yf.

10. The process of any preceding claim, which is conducted in the gas phase.

## Patentansprüche

1. Verfahren zur Herstellung von 2,3,3,3-Tetrafluor-1-propen (HFO-1234yf), bei dem man CF₃Cl mit CH₃CF₂Cl in Berührung bringt, wobei der Schritt des Inberührungbringens in Gegenwart eines auf Metall basierenden Katalysators durchgeführt wird.

2. Verfahren nach Anspruch 1, bei dem das Molverhältnis von CH₃CF₂Cl: CF₃Cl im Bereich von 1:1 bis 4:1 liegt.

3. Verfahren nach Anspruch 2, bei dem das Molverhältnis von CH₃CF₂Cl:CF₃Cl im Bereich von 1,5:1 bis 2,5:1 liegt.

4. Verfahren nach einem der vorhergehenden Ansprüche, bei dem man den auf Metall basierenden Katalysator aus auf Nickel basierenden Katalysatoren, auf Palladium basierenden Katalysatoren, auf Antimon basierenden Katalysatoren, auf Aluminium basierenden Katalysatoren und auf Eisen basierenden Katalysatoren auswählt.

5. Verfahren nach einem der vorhergehenden Ansprüche, bei dem im Schritt des Inberührungbringens mindestens 50% des CH₃CF₂Cl umgewandelt werden.

6. Verfahren nach einem der vorhergehenden Ansprüche, bei dem im Schritt des Inberührungbringens mindestens 90% des CH₃CF₂Cl umgewandelt werden.

7. Verfahren nach einem der vorhergehenden Ansprüche, bei dem im Schritt des Inberührungbringens ein Reaktionsprodukt mit einer Selektivität für HFO-1234yf von mindestens 20% anfällt.

8. Verfahren nach Anspruch 7, bei dem im Schritt des Inberührungbringens ein Reaktionsprodukt mit einer Selektivität für ein oder mehrere Tetrafluorpropene von mindestens 70% anfällt.

9. Verfahren nach einem der vorhergehenden Ansprüche, bei dem im Schritt des Inberührungbringens ein Reaktionsprodukt mit einer Selektivität für HFO-1234yf von mindestens 70% anfällt.

10. Verfahren nach einem der vorhergehenden Ansprüche, das man in der Gasphase durchführt.

## Revendications

1. Procédé de préparation de 2,3,3,3-tétrafluoro-1-propène (HFO-1234yf), le procédé comprenant la mise en contact du CF₃Cl avec le CH₃CF₂Cl, ladite étape de mise en contact étant effectuée en présence d'un catalyseur à base de métaux.

2. Procédé selon la revendication 1, **caractérisé en ce que** le rapport molaire CH₃CF₂Cl:CF₃Cl est dans la gamme de 1:1 à 4:1.

3. Procédé selon la revendication 2, **caractérisé en ce que** le rapport molaire CH₃CF₂Cl:CF₃Cl est dans la gamme de 1,5:1 à 2,5:1.

4. Procédé selon l'une quelconque des revendications précédentes, **caractérisé en ce que** le catalyseur à base de métaux est choisi parmi les catalyseurs à base de nickel, les catalyseurs à base de palladium, les catalyseurs à base d'antimoine, les catalyseurs à base de l'aluminium et les catalyseurs à base de fer.

5. Procédé selon l'une quelconque des revendications précédentes, **caractérisé en ce que** ladite étape de mise en contact est efficace pour transformer au moins 50 % dudit CH₃CF₂Cl.

6. Procédé selon l'une quelconque des revendications précédentes, **caractérisé en ce que** ladite étape de mise en contact est efficace pour transformer au moins 90 % dudit CH₃CF₂Cl.

7. Procédé selon l'une quelconque des revendications précédentes, **caractérisé en ce que** ladite étape de mise en contact est efficace pour produire un produit de réaction ayant une sélectivité d'au moins 20 % vis-à-vis de HFO-1234yf.

8. Procédé selon la revendication 7, **caractérisé en ce que** ladite étape de mise en contact est efficace pour produire un produit de réaction ayant une sélectivité d'au moins 70 % vis-à-vis d'un ou de plusieurs tétrafluoropropènes.

9. Procédé selon l'une quelconque des revendications précédentes, **caractérisé en ce que** ladite étape de mise en contact est efficace pour produire un produit de réaction ayant une sélectivité d'au moins 70 % vis-à-vis de HFO-1234yf.

10. Procédé selon l'une quelconque des revendications précédentes, **caractérisé en ce qu'**il est effectué en phase gazeuse.
